# EUROPÄISCHE PATENTANMELDUNG

(11) **EP 0 976 443 A1**
(43) Veröffentlichungstag der Anmeldung: **02.02.2000**
(21) Anmeldenummer: 99114049.2
(22) Anmeldetag: 20.07.1999
(51) Int. Cl.: B01F 13/00, A61F 2/46, A61C 5/06

(54) **Verfahren zum Mischen von Calcium-Phosphat-Zementen**

(30) Priorität: 31.07.1998 DE 19834504
(71) Anmelder: MERCK PATENT GmbH, 64293 Darmstadt (DE)
(72) Erfinder: Wenz, Robert, Dr., 61206 Wöllstadt (DE)

(57) **Zusammenfassung**

Die Erfindung betrifft ein Verfahren zum Mischen von Calcium-Phosphat-Zement, bestehend aus den Reaktionsschritten:
- Injizieren einer Flüssigkeit in eine mit Pulver beladene Kapsel
- Einspannen der Kapsel in ein Mischgerät
- Mischen mit voreingestellter Mischzeit und
- Entfernen des Produktes aus der Kapsel.

## Beschreibung

Die Erfindung betrifft ein Verfahren zum Mischen von Calcium-Phosphat-Zementen, die in der Medizin als Knochenersatzwerkstoff Anwendung finden.

Das effiziente Mischen von Pulver und Flüssigkeit bei Calcium-Phosphat-Zementen stellt deshalb ein Problem dar, weil relativ große Pulvermengen mit relativ kleinen Volumina Flüssigkeit zur Reaktion gebracht werden müssen. Das Verhältnis Pulver zu Flüssigkeit, das zu vermischen ist, beträgt bei Biozement D 0.3 und bei α-BSM (=bone substitute material; BIOBONE®, Fa. Etex Corp., USA) 0.8.

Nach der WO 98/15314 wird z.B. α-BSM angemischt, indem das Pulver, welches in einem elastischen Kunststoffbeutel abgefüllt ist, nach Zuspritzen der entsprechenden Flüssigkeitsmenge, mindestens 1 Minute lang sorgfältig geknetet wird. Hierdurch soll eine ausreichende Benetzung der Reaktionspartikel gewährleistet werden. Weil das Kneten individuell verschieden ist, kann daraus eine mehr oder weniger quantitativ durchmischte Paste resultieren. Ein weiterer Nachteil besteht darin, daß zuweilen nicht benetztes Pulver im Kunststoffbeutel zurückbleibt. Dies bedeutet aber, daß letztendlich der geforderte Quotient Pulver zu Flüssigkeit = 0.8 nicht erreicht wird.

Aufgabe der vorliegenden Erfindung ist es, nunmehr ein Verfahren zum Mischen von Calcium-Phosphat-Zement zur Verfügung zu stellen, welches die o.g. Nachteile des Standes der Technik vermeidet.

Diese Aufgabe wird überraschend einfach durch ein Verfahren zum Mischen von Calcium-Phosphat-Zement gelöst, indem man eine Flüssigkeit, vorzugsweise unter sterilen Bedingungen, in eine mit Pulver beladene Kapsel injiziert, die Kapsel, vorzugsweise im Peel-off-Beutel, in ein Mischgerät einspannt, bei voreingestellter Mischzeit mischt und das fertige Produkt anschließend aus der Kapsel entfernt.

Ein weiterer Vorteil der vorliegenden Erfindung ist, daß durch die Benutzung einer Kapsel (APLICAP® -System der Fa. Espe; EP-291733), in welche die Flüssigkeit injiziert wird und durch Einspannen dieser Kapsel in ein handelsübliches Dentalmischgerät (CAPMIX® der Fa. Espe; EP-291733) mit voreingestellter Mischzeit, Pulver und Flüssigkeit der Calcium-Phosphat-Zemente stets gleichbleibend und effizient, ohne Zurückbleiben von unbenetztem Pulver, gemischt werden.
Bevorzugt ist dabei, daß der Mischvorgang unter sterilen Bedingungen erfolgt. Dies geschieht durch Aktivieren der Kapsel mit steriler Umverpackung, wobei die sterile Umverpackung als Peel-off-Beutel (=Aufreißverpackung) geschaffen ist. Dies hat den Vorteil, daß während des Aktivierungsvorganges die sterile Umverpackung nicht beschädigt wird, so daß auch nach dem Mischvorgang die Sterilität der Kapsel gewahrt bleibt.

Ein weiterer Vorteil dieses Mischverfahrens ist der, daß die Anmischzeit, im Beispiel von α-BSM, auf 15 Sekunden und weniger gekürzt werden kann und daß Festigkeiten von 6 bis 8 MPa, vorzugsweise von 7.6 MPa, des ausgehärteten Materials im Vergleich zur Handmischung (2-4 MPa) erzielt werden. Der gleiche Effekt ist auch bei Biozement D zu beobachten.

Eine weitere Aufgabe besteht darin, ein Verfahren zum Mischen von Calcium-Phosphat-Zement bereitzustellen, durch das es möglich ist, eine injizierbare Paste in einer Spritze unter sterilen Bedingungen in einem Blister (= festes Formteil) abgepackt zur Verfügung zu stellen.

Diese Aufgabe wird durch ein Verfahren zum Mischen von Calcium-Phosphat-Zement gelöst, bei dem eine mit Pulver und der entsprechenden Flüssigkeitsmenge beladene und beidseitig verschlossene Spritze mittels eines handelsüblichen Dentalmischgerätes (CAPMIX®) vertikal oder unter Verwendung eines Rotationsmischers (ROTOMIX® der Fa. Espe) rotierend durchmischt wird. Dadurch ist es möglich nach der Mischzeit eine injizierbare Paste nach Lösen der Arretierung am Kolbenboden in einem Arbeitsgang injektionsbereit zur Verfügung zu stellen. Vorzugsweise kann die Spritze in einem sterilisierbaren Blister abgepackt werden, der paßgenau auf eine Ausnehmung oder Vorwölbung am Aufnehmer des Mischgerätes aufgesteckt wird und damit gleichzeitig ein steriles Umfeld für die Spritze gegenüber dem nicht sterilen Mischgerät garantiert.

In den folgenden Beispielen sind Ausführungsformen der Calcium-Phosphat-Zemente, die nach dem erfindungsgemäßen Mischverfahren hergestellt wurden, näher erläutert:
Der handelsübliche Biocement D besteht aus einem Gemisch von α-Tricalciumphosphat, Monotit, Calciumcarbonat und PHA (= Hydroxylapatit).

### Herstellung von Calcium-Phosphat-Zementen

### Beispiel 1 mit α-BSM

Bei Raumtemperatur werden 3.6 g Calcium-Phosphat-Pulver in einer Kapsel (APLICAP®) unter sterilen Bedingungen vorgegeben und 0.8 ml einer wäßrigen Flüssigkeit z.B. NaH₂PO₄ oder Na₂HPO₄ oder einer organischen Flüssigkeit wie Polyacrylsäure zugegeben. Dies geschieht durch Aktivieren der Kapsel mit steriler Umverpackung, wobei die sterile Umverpackung als Peel-off-Beutel (=Aufreißverpackung) geschaffen ist. Dies hat den Vorteil, daß während des Aktivierungsvorganges die sterile Umverpackung nicht beschädigt wird, so daß auch nach dem Mischvorgang die Sterilität der Kapsel gewahrt bleibt.
Die Kapsel wird in das Mischgerät für Dentalmaterialien CAPMlX® eingespannt und bei einer Mischfrequenz von ca. 4450 Schwingungen pro Minute ca. 15 Sek. lang gemischt. Nach Ablauf der Mischzeit kann die Kapsel mit dem fertigen α-BSM entnommen werden.
Der so hergestellte α-BSM (= BIOBONE®, Fa. Etex Corp., USA) besitzt nach der Aushärtung eine Festigkeit von etwa 7.6 MPa.

### Beispiel 2 mit Biocement D

Eine mit 1 g Calcium-Phosphat beladene und beidseitig verschlossene, sterile Spritze wird mit 0.3 ml Flüssigkeit (siehe Beispiel 1) durch einen am Luer-Konus befindlichen Stopfen injiziert. Die so beladene Spritze wird in das CAPMIX-Mischgerät oder alternativ in das Rotationsmischgerät (ROTOMIX®) eingespannt und ca. 15 Sek. lang vertikal bzw. rotierend durchmischt. Nach dem Mischvorgang und dem Lösen der Arretierung am Kolbenboden steht somit eine Paste injektionsbereit zur Verfügung.
Alternativ kann die sterile Spritze in einem sterilisierbaren Blister abgepackt werden, der paßgenau auf eine Ausnehmung oder Vorwölbung am Aufnehmer des CAPMIX-Mischgerätes aufgesteckt wird. Hierdurch wird ein steriles Umfeld für die Spritze gegenüber dem nicht sterilen Mischgerät gewährleistet.
Die Festigkeit des so hergestellten Biocementes D ist der des α-BSM vergleichbar.

## Patentansprüche

1. Verfahren zum Mischen von Calcium-Phosphat-Zement, bestehend aus den Reaktionsschritten:
- Injizieren einer Flüssigkeit in eine mit Pulver beladene Kapsel
- Einspannen der Kapsel in ein Mischgerät
- Mischen mit voreingestellter Mischzeit und
- Entfernen des Produktes aus der Kapsel.

2. Verfahren gemäß Anspruch 1, dadurch gekennzeichnet, daß
- das Injizieren einer Flüssigkeit in eine mit Pulver beladene Kapsel unter sterilen Bedingungen und
- das Einspannen der Kapsel in ein Mischgerät im Peel-off-Beutel erfolgt.

3. Verfahren gemäß Anspruch 1 und/oder 2, dadurch gekennzeichnet, daß Mischungen konstanter Qualität entstehen.

4. Verfahren nach den Ansprüchen 1 bis 3, dadurch gekennzeichnet, daß die Anmischzeit zur Herstellung von α-BSM maximal 15 Sekunden beträgt.

5. Verfahren nach einen der Ansprüche 1 bis 4, dadurch gekennzeichnet, daß bei ausgehärtetem α-BSM Festigkeiten von 6 bis 8 MPa resultieren.

6. Verfahren zum Mischen von Calcium-Phosphat-Zement, dadurch gekennzeichnet, daß eine mit Pulver und der entsprechenden Flüssigkeitsmenge beladene Spritze mittels eines Mischgerätes vertikal durchmischt wird.

7. Verfahren zum Mischen von Calcium-Phosphat-Zement gemäß Anspruch 6, dadurch gekennzeichnet, daß die beladene sterile Spritze unter Verwendung eines Rotationsmischers rotierend durchmischt wird.

8. Verfahren nach einem der Ansprüche 6 und/oder 7, dadurch gekennzeichnet, daß die beladene Spritze nach dem Mischen in einen sterilen Blister abgepackt wird.

9. Verfahren nach einem der Ansprüche 6 bis 8, dadurch gekennzeichnet, daß die beladene Spritze mit dem sterilen Blister paßgenau auf eine Ausnehmung oder Verwölbung am Aufnehmer des Mischgerätes aufgesteckt wird.

10. Verwendung der nach dem Verfahren eines der vorhergehenden Ansprüche hergestellten Calcium-Phosphat-Zemente zur Herstellung von Knochenersatzwerkstoffen, insbesondere in der Medizin.
